(19) Europäisches Patentamt
European Patent Office
Office européen des brevets

(11) Publication number: **0 160 451 B1**

(12) # EUROPEAN PATENT SPECIFICATION

(45) Date of publication of patent specification: **28.08.91**

(51) Int. Cl.5: **C07D 401/12**, A61K 31/44, C07B 57/00

(21) Application number: **85302666.4**

(22) Date of filing: **16.04.85**

(54) **Dihydropyridine-3,5-dicarboxylic acid ester derivative isomers, their preparation, and pharmaceutical compositions containing them.**

(30) Priority: **16.04.84 JP 75998/84**
**04.06.84 JP 114098/84**
**07.08.84 JP 165793/84**

(43) Date of publication of application:
**06.11.85 Bulletin 85/45**

(45) Publication of the grant of the patent:
**28.08.91 Bulletin 91/35**

(84) Designated Contracting States:
**AT BE CH DE FR GB IT LI LU NL SE**

(56) References cited:
**GB-A- 2 014 134**

**JAPAN, J. PHARMACOL. 32, 665 (1982)**

(73) Proprietor: **YAMANOUCHI PHARMACEUTICAL CO. LTD.**
**No. 5-1 Nihonbashi-Honcho, 2-chome Chuo-ku**
**Tokyo(JP)**

(72) Inventor: **Tamazawa, Kazuharu**
**1013-2, Oaza Kaminoda Shiraoka-cho**
**Minamisaitama-gun Saitama(JP)**
Inventor: **Kojima, Tadao**
**No. 1086-2, Oaza Sanegaya Shiraoka-cho**
**Minamisaitama-gun Saitama(JP)**
Inventor: **Arima, Hideki**
**No. 4-5-210, Nobidome 3-chome**
**Higashikurume-shi Tokyo(JP)**
Inventor: **Murakami, Yukiyasu**
**No. 306-3, Ohmaki**
**Urawa-shi Saitama(JP)**
Inventor: **Isomura, Yasuo**
**No. 12-11-919, Sukai-Haitsu Nishiogu**
**4-chome**
**Arakawa-ku Tokyo(JP)**

EP 0 160 451 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Okada, Minoru**
**No. 10-20, Higashiooi 6-chome**
**Shinagawa-ku Tokyo(JP)**
Inventor: **Takanobu, Kiyoshi**
**No. 1142-1, Hongo-cho**
**Omiya-shi Saitama(JP)**
Inventor: **Takenaka, Toichi**
**No. 27 H-1108, Nakadai 3-chome**
**Itabashi-ku Tokyo(JP)**

(74) Representative: **Geering, Keith Edwin et al**
**REDDIE & GROSE 16 Theobalds Road**
**London WC1X 8PL(GB)**

## Description

This invention relates to a diastereoisomer of YM-09730 (defined below),its dextro-rotatory optical isomer,and pharmaceutically acceptable acid addition salts of these compounds; it further relates to production of these compounds and also to medicaments using these compounds as effective component.

YM-09730 is a dihydropyridine-3,5-dicarboxylic acid ester derivative shown by the following chemical structure, the chemical name of which is 2,6-dimethyl-4-(3-nitrophenyl)-1,4-dihydropyridine-3,5-dicarboxylic acid 3-(1-benzylpyrrolidin -3-yl)ester 5-methyl ester.

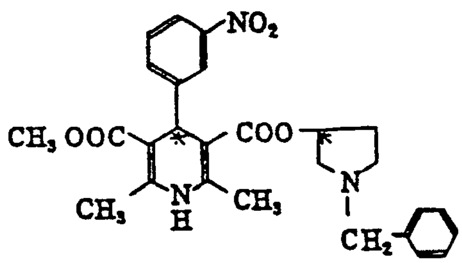

YM-09730 was first synthesized by us, and has vasodilating and hypotensive activity of good durability (U.S.Patent No.4,22O,649); U.K.Patent No.2,O14,134).

YM-O973O has two asymmteric carbon atoms, but there is no description of isomers in the above-mentioned patents and the existence of isomers has not previously been confirmed. A pair of diasetereoisomers differ in absolute value of rotatory power and in many physical and chemical properties (unlike general optical antipodes) and hence if these properties are not clarified the isomers cannot be obtained separately on the basis of these property differences.

We have now succeeded in separating diastereoisomers A and B of YM-O973O, and their individual optical isomers, and have discovered that"isomer A" (which term is applied equally herein to the diastereoisomer A and to the dextro-rotatory optical isomer there of) has greater specific pharmaceutical effects as compared to "isomer B" (which term is applied equally herein to the diastereoisomer B and to optical isomers thereof) or to a mixture (YM-O973O) of diastereoisomers A and B. The melting point of the hydrochloride of the diastereoisomer A is 2OO to 2O6°C (decomp.) and the melting point of the hydrochloride of the dextro-rotatory optical isomer thereof is 223 to 23O°C (decomp.). Accordingly, subjects of this invention are diastereoisomer A of YM-O973O and its dextro-rotatory isomer (identified by the melting points of their hydrochlorides) and also pharmaceutically acceptable acid addition salts thereof.

Suitable pharmaceutically acceptable acid addition salts in this invention include malonates, oxalates, p-nitrobenzoates, 2-ketoglutarates, maleates, 1-malates, hydrochlorides, sulfates, p-toluenesulfonates, phosphates and succinates.

In the pharmacological experiments described below isomer A of this invention and pharmaceutically acceptable acid addition salts thereof gave a coronary blood flow increase 15 to 38 times that of diastereoisomer B and 14 to 35 times that of an equal mixture of diastereoisomers A and B on direct administration into the coronary arteries, which shows a high affinity of isomer A of this invention for the coronary arteries.

On the other hand, isomer B showed almost the same effect as the mixture of the isomers. This means that the pharmacological action of YM-09730 is not a simple average of the actions of the isomers. It is new pharmacological knowledge that isomer A and the pharmaceutically acceptable acid addition salts thereof have a high affinity for coronary arteries and this increases the possibility of the utilization of the compounds of this invention as medicaments.

The vasodilatory 1,4-dihydropyridine derivative nicardipine (e.g. US-A-3985758 of 10th October 1976) has a single asymmetric carbon atom. Japan J. Pharmacol.32, 665 (1982) describes resolution of the two optical isomers but the difference in activity of the more active isomer over the racemate is at most threefold.

The production of diastereoisomer A and pharmaceutically acceptable acid addition salts thereof may be as follows:

I. Production of diastereoisomer A

YM-O973O can be prepared by Hantzch's synthesis (Ann. Chim., 215, 1(1882)) as in Reference

Example 1 below, giving a 1:1 mixture of diastereoisomer A and diastereoisomer B.

Diastereoisomer A could then be obtained as follows:

(a) The mixture of the diastereoisomers was subjected to column chromatography using silica gel as the carrier and a mixed solution of ethyl acetate and acetic acid as the eluent,and diastereoisomer A was obtained from the initial eluate and diastereoisomer B from the later eluate.

As silica gel carrier for the column chromatography, any silica gel conventional for the purpose can be used. Also, there is no particular restriction on the mixing ratio of ethyl acetate and acetic acid used as the eluent,but usually the mixed solvent containing a small amount of acetic acid is used. The mixing ratio is e.g. 30 to 50 v/v of ethyl acetate and about 1 to 10 v/v of acetic acid and if the content of acetic acid is lowered, the eluting time of the desired compound is prolonged. The elution rate and the processing temperature can be selected as desired.

(b) Another process, produces an acid addition salt of diastereoisomer A by converting the mixture of the diastereoisomers to the acid addition salts and subjecting the mixture of the salts to fractional recrystallization.

Suitable acid addition salts for use in this process are e.g. malonate, p-nitrobenzoate , maleate , etc.

These acid addition salts are crystalline salts and the solubility in an organic solvent differs between diastereoisomer A and diastereoisomer B, thus allowing production of diasetereoisomer A by fractional recrystallization. A particularly suitable acid addition salt is malonate . In the case of using the malonate , crystals having a very high content of diastereoisomer A can be obtained by one recrystallization. Suitable solvents for use in the process are e.g. methanol, ethanol, acetone, acetonitrile, etc.

The acid addition salt of diastereoisomer A of YM-09730 obtained by the above-described process can be acid-exchanged into another desired acid addition salt by conversion to free form and reaction with other acid.

II. Production of dextro-rotatory optical isomer A:

(a) The dextro-rotatory optical isomer of diastereoisomer A can be obtained by reacting the mixed free bases of diastereoisomers A and B or the free base of the acid addition salt of diastereoisomer A of YM-09730 obtained by the process I above with L-(- )-malic acid and then subjecting the product to optical resolution in conventional manner as described hereinafter.

(b) In a more preferred process the dextro-rotatory optical isomer of diastereoisomer A is separated from admixture(of formula 1 below) with the levo-rotatory optical isomer of diastereoisomer B

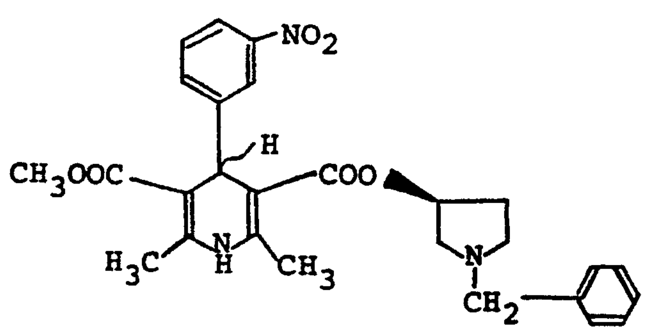

(I)

(wherein the wavelike bond means an α-bond or β-bond,

the bond shown by the dense arrow is a β-bond, and the bond at the 3-position of the pyrroldine ring is a specific β-bond).

The raw material compound (I) can be produced

(i) by reacting m-nitrobenzaldehyde shown by formula (II)

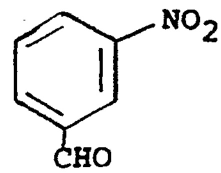

(II),

(S)-3-acetoacetoxy-1-benzylpyrrolidine shown by formula (III)

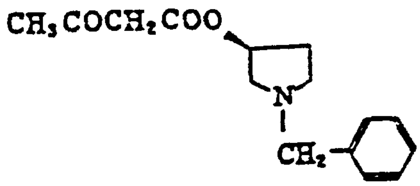

(III)

and 3-aminocrotonic acid methyl ester shown by formula (IV)

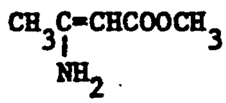

(IV);

(ii) by reacting m-nitrobenzaldehyde shown by formula (II) above, acetoacetic acid methyl ester shown by formula (V)

$CH_3COCH_2COOCH_3$ (V)

and (S)-3-(3-aminocrotonoyloxy)-1-benzylpyrrolidine show by formula (VI)

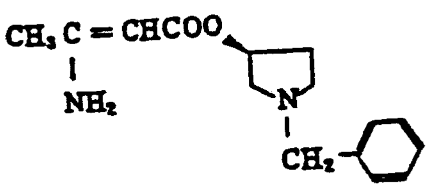

(VI);

(iii) reacting (s)-1-benzyl-3-[2-(m-nitrobenzylidene)-acetoacetoxy]pyrrolidine shown by formula (VII)

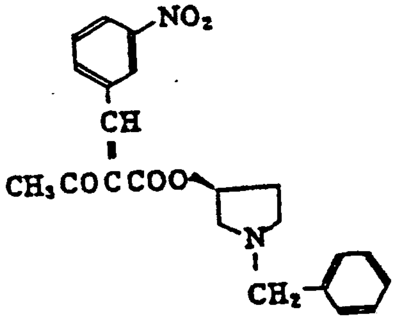

(VII),

obtained by previously reacting m-nitrobenzaldehyde (II) above and (S)-3-acetoacetoxy-1-benzylpyrrolidine (III) above and 3-aminocrotonic acid methyl ester (IV) above; or

(iv) reacting 2-(m-nitrobenzylidene)acetoacetic acid methyl ester shown by formula (VIII)

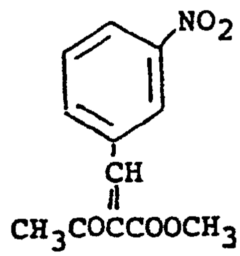

(VIII)

obtained by previously reacting m-nitrobenzaldehyde (II) and acetoacetic acid methyl ester (V) and (S)-3-(3-aminocrotonoyloxy)-1-benzylpyrrolidine (VI).

These reactions proceed without use of solvent but it is advantageous to perform the reaction in a solvent which does not take part in the reaction, such as an alcohol, dioxane, dimethylformamide, dimethylsulfoxide, acetonitrile, water, etc. The reaction is performed by heating a mixture of almost equimolar amounts of each component.

The compound (IV) or (VI) above can be obtained by reacting the compound (V) or (III) with ammonium acetate and acetic acid in benzene and azeotropically dehydrating the product. The compound (IV) or (VI) thus obtained is supplied to reaction (i) or (ii) described above after being isolated or without being isolated from the reaction mixture.

The compound (VII) or (VIII) which is the reaction product in the first step of process (iii) or (iv) can be supplied to the last step after being isolated once or without being isolated.

The mixture of the dextro-rotatory optical isomer of diastereoisomer A and the levo-rotatory optical isomer of diastereoisomer B thus obtained is subjected to column chromatography using silica gel as the carrier and a mixture of ethyl acetate and acetic acid as the eluent to separate the dextro-rotatory optical isomer; or the mixture described above is reacted with L-(-)-malic acid to form a mixture of the L-(-)-malate of the dextro-rotatory optical isomer of diastereoisomer A and the L-(-)-malate of the levo-rotatory optical isomer of diastereoisomer B,and by fractionally recrystallizing the mixture the L-(-)-malate of the dextro-rotatory optical isomer of diastereoisomer A can be obtained.

In the separation by column chromatography, the dextro-rotatory optical isomer of diastereoisomer A is obtained from the first eluate and the levo-rotatory optical isomer of diastereoisomer B can be obtained from the later eluate.

As silica gel carrier, any silca gel which is generally used for column chromatography can be used. There is no particular restriction on the mixing ratio of ethyl acetate and acetic acid for use as the eluent but the mixed solvent containing a small amount of acetic acid is preferred. It is advantageous that the mixing ratio is 30 to 50 v/v of ethyl acetate and about 1 to 10 v/v of acetic acid and when the content of acetic acid is further lowered, the eluting time for the desired compound is prolonged. The eluting time and processing temperature can be selected as desired.

The process of using L-(-) malic acid can also be utilized for the separation of the dextro-rotatory optical isomer of diastereoisomer A by recrystallization since the L-(-)-malate of the dextro-rotatory optical isomer of diastereoisomer A is crystalline. Suitable solvents for use in the fractional recrystallization are e.g. methanol, ethanol, acetone, acetonitrile, etc.

The L-(-)-malate of the dextro-rotatory optical isomer of diastereoisomer A thus obtained can be used as a medicament, but can be converted to the acetate or other suitable salt, if necessary, by treating with base to form the free compound and treating this with appropriate acid.

By hydrolyzing the levo-rotatory optical isomer of diastereoisomer B separated in the above process, (S)-(-)-1-benzyl-3-hydroxypyrrolidine can be recovered and the pyrrolidine can be reused as raw material for producing the compound of formula (I).

The pharmacological activities,acute toxicity and clinical doses of compounds according to the invention are illustrated by the following tests.

(1) Coronary vasodilating effect in anesthetized dogs:

In open-chest dogs anesthetized with 30 mg/kg i.v. of pentobarbital sodium, arterial blood from the carotid artery was led to the circumflex branch of the left coronary artery by extracorporeal loop. A servocontrolled pump(model 1215D, Harvard Apparatus) was incorporated in the circuit to maintain a

constant perfusion pressure of 120 mmHg by means of a pump controller (SCS-22, Data Graph Co., Tokuya Tukada et al., Folia Pharmacol. Japon, 74, 59p, 1978). An electromagnetic flow probe of extracorporeal type (MF-25, Nihon Koden) was also inserted in the circuit to record coronary blood flow. A dose of 1 μg of test compound was administered directly into the coronary artery and then coronary blood flow was monitored until the blood flow returned to the pretreatment value. The area under the curve for percent increase in coronary blood flow after the intracoronary injection was calculated and used as an index of the overall increase in coronary blood flow. Results are shown in Table 1.

Table 1. Coronary vasodilating activity in anesthetized dogs

| | Overall increase in coronary blood flow at 1 μg (%, min.) | Duration of action |
|---|---|---|
| Diastereoisomer A(dl form) hydrochloride | 1816 ± 377 | 60 |
| Diastereoisomer A (d form) hydrochloride | 4559 ± 894 | 120 |
| Diastereoisomer A (l form) hydrochloride | 120 ± 21 | 5 |
| Diastereoisomer B (dl form) hydrochloride | 120 ± 28 | 5 |
| 1:1 mixture of the hydrochlorides of diastereoisomers A (dl form) and B (dl form) | 129 ± 47 | 10 |

The overall increase in coronary blood flow caused by the isomer A hydrochloride was approximately 15 to 38 times higher than those caused by identical doses of the isomer B hydrochloride and the equal mixture of A and B isomers, indicating that the isomer A hydrochloride possesses a high affinity for the coronary artery. Furthermore, duration of the coronary vasodilating activity of the isomer A hydrochloride was clearly longer than those of the isomer B hydrochloride and the mixture of A and B isomers. Such a high affinity for the coronary artery and long durability indicate that the isomer A hydrochloride is useful for the treatment of coronary artery disease such as angina pectoris.

(2) Hypotensive effect in anesthetized rats:

Blood pressure was measured in urethane-anesthetized rats. Test compound was intravenously administered in an increasing dose manner at interval of 20 minutes. The dose of each compound required to lower mean blood pressure by 30 mmHg (ED 30 mmHg) was calculated from the dose-response curves and is shown in Table 2.

Table 2. Hypotensive activity in anesthetized rats

| | ED30 mmHg (mg/kg i.v.) |
|---|---|
| Diastereoisomer A (dl form) hydrochloride | 0.002 |
| Diastereoisomer B (dl form) hydrochloride | 0.14 |

As can be seen in Table 2, the hypotensive activity of the diastereoisomer A hydrochloride was about 70 times more potent than that of the diastereoisomer B hydrochloride.

(3) Acute toxicity in mice:

Six weeks old and male ICR mice weighing 27 to 29 g were used. Test compound was suspended in 0.5% methylcellulose solution and administered orally to mice. The $LD_{50}$ values of the compounds were calculated by the method of Litchfield & Wilcoxon (Journal of Pharmacol. & Exp. Therap., 96, 99-113, 1949) and are shown in Table 3.

Table 3. Acute toxicity in mice

| | $LD_{50}$ mg/kg p.o. |
|---|---|
| Diastereoisomer A (dl form) hydrochloride | 295 (242-360) |
| Diastereoisomer A (d form) hydrochloride | 190 (158-228) |

(4) Clinical doses:

The clinical doses of the compounds of this invention depend on the body weight and the state of the disease of the patient. The optimal doses are usually 0.1 to 2 mg for intravenous injection and 5-20 mg once or twice a day for oral administration.

The compounds of this invention, their production, and the medicaments using them are illustrated by the following Examples and Formulation Examples. The production of a mixture of diastereoisomer A and diastereoisomer B which is used as a raw material is illustrated in Reference Example 1 and the production of (S)-(-)-1-benzyl-3-hydroxypyrrolidine which is also used as a raw material is illustrated in Reference Examples 2 - 4.

Reference Example 1

In 5 ml of isopropanol were dissolved 1.51 g (0.01 mole) of 3-nitrobenzaldehyde, 2.61 g (0.01 mole) of 1-benzyl-3-acetoacetoxypyrrolidine, and 1.15 g (0.01 mole) of 3-aminocrotonic acid methyl ester and the solution thus obtained was refluxed for 8 hours. Then the solvent was distilled off under reduced pressure, the residue thus formed was dissolved in chloroform, and the solution was washed in succession with dilute hydrochloric acid, water, and saturated aqueous sodium hydrogencarbonate solution and dried over anhydrous magnesium sulfate. Then the solvent was distilled off under reduced pressure to provide 4.91 g of 2,6-dimethyl-4-(3-nitrophenyl)-1,4-dihydropyridine-3,5-dicarboxylic acid 3-(1-benzylpyrrolidin-3-yl)ester 5-methyl ester in caramel form.

The ratio of the diastereoisomers thus obtained as the crude base was analyzed by reverse phase ion-pair high-performance liquid chromatography (HPLC) under the following conditions:

Column: 4.6 mm x 300 mm packed with Nucleosil® 5 $C_{18}$, column temperature: 30° C, mobile phase: acetonitrile- 0.05 mole potassium dihydrogenphosphate (pH 3) (20:80 v/v) containing tetra-n-pentylammonium bromide (3 mmole) as counter ion, Flow rate: 0.9 ml/min., Monitor: Ultraviolet detector (λ 254 nm).

The ratio of diastereoisomer A (retention time 28 min.): diastereoisomer B (retention time 29 min.) was 1 : 1.

By nuclear magnetic resonance analysis, diastereoisomer A and diastereoisomer B were confirmed to have different chemical shifts with respect to the methylene protons of the N-benzyl group of YM-09730. By measurement in heavy methanol $d_4$ ($CD_3OD$) with Jeol NMR-spectrometer FX-90Q, isomer A and isomer B show the singlet signals corresponding to two protons at 4.40 ppm and 4.30 ppm, respectively.

Reference Example 2

(1) In 66 ml of acetone were dissolved 17.7 g of dl-1-benzyl-3-hydroxypyrrolidine and 15.2 g of D-(-)-mandelic acid with heating and the solution was allowed to stand overnight at 4° C to deposit crystals; 8.5 g of the crystals thus deposited were collected and recrystallized from 26 ml of acetone to provide 5.1 g of D-(-)-mandelate of (S)-(-)-1-benzyl-3-hydroxypyrrolidine. The specific rotation $[\alpha]_D^{20}$ was -45.5° C (c = 1, methanol). when the recrystallization was repeated, the specific rotation was not changed. The melting point was 101-102°C.

By nuclear magnetic resonance analysis the signal of N-$\underline{CH_2}$-Ph (of the thus obtained mandelate of (S)-(-)-form) was observed at 4.03 ppm (singlet, 2H). The signal of an AB-type quartet (J = 12.5 Hz) at 4.01 ppm of the (R)-(-) form was not observed.

(2) In 50 ml of chloroform was dissolved 22 g of D-(-)-mandelate of (S)-(-)-1-benzyl-3-hydroxypyrrolidine and the chloroform solution thus formed was washed with a solution of 14.4 g of sodium carbonate in 90 ml of water and dried over anhydrous magnesium sulfate. Then, after distilling off chloroform, the residue was distilled under reduced pressure to provide 11.5 g of S-(-)-1-benzyl-3-hydroxypyrrolidine. The boiling point was 109°C/0.65 mmHg and$[\alpha]_D^{20}$ was -3.77° (c = 5, methanol).

Reference Example 3

By reacting 75 g of (S)-(-)-malic acid and 75 ml of benzylamine for 3 hours at 170°C, 52.7 g of (S)-(-)-1-benzyl-3-hydroxy-succinimide (melting point 99-101° C, specific rotation$[\alpha]_D^{20}$ -51.1°, (c= 1, methanol)) was obtained. In 340 ml of anhydrous tetrahydrofuran was suspended 9.73 g of lithiumaluminum hydride and a solution of 20.5 g of the above-described imide in 200 ml of anhydrous tetrahydrofuran was added dropwise to the suspension under ice-cooling. After refluxing the mixture for 3 hours, 100 g of sodium sulfate decahydrate was added to the mixture under ice-cooling and the resultant mixture was stirred overnight under ice-cooling. Insoluble solids wore filtered off , the solvent was evaporated from the filtrate under reduced pressure, and the residue was distilled under reduced pressure to provide 13.8 g of (S)-(-)-1-benzyl-3-hydroxypyrrolidine having a boiling point of 109 to 115°C/0.8 mmHg and specific rotation $[\alpha]_D^{20}$ of -3.0° (c=5, methanol). It was confirmed,by proton nuclear magnetic resonance analysis of the 3-position using shift reagent Eu-TFMC(III) that the (S)-(-)-form obtained above contained 10% R-(+)-1-benzyl-3-hydroxypyrrolidine. The product was converted to the D-(-)-mandelate as in Reference Example 2. The mandelate $[\alpha]_D^{20}$ -45.2° (c=1, MeOH),obtained by recrystallizing from 3 times by volume of ethanol and then 6 times by volume of ethanol-toluene (1 : 5 v/v), was treated with chloroform and a solution of aqueous sodium carbonate as in Preference Example 2 to give 8.6 g of (S)-(-)-1-benzyl-3-hydroxypyrrolidine; boiling point 115 to 120° C/1.2 to 1.5 mmHg, $[\alpha]_D^{20}$ .-3.77° (c=5 methanol).

Reference Example 4

To 50 ml of 9-borabicyclo[3,3,1]nonane (0.5M tetrahydrofuran solution) was added 3.4 g of 2-(-)-pinene and the mixture was stirred for 5 hours at 60°C. After cooling the mixture to room temperature, 1.75 g of 1-benzyl-3-pyrrolidinone was added thereto. After stirring the mixture for 4 days at room temperature, 1.3 ml of acetaldehyde was added thereto at 0°C. Then the solvent was distilled off under reduced pressure and 20 ml of ether was added to the residue. After cooling the mixture to 0°C, 1.5 ml of 2-aminoethanol was added thereto and the resultant mixture was stirred. Precipitates thus formed were filtered off . The ether solution recovered as the filtrate was extracted with 1 N hydrochloric acid and the hydrochloric acid layer

thus formed was alkalified with sodium carbonate and extracted with dichloromethane. The extract thus formed was dried by anhydrous magnesium sulfate and concentrated to provide 1.1 g of a crude product. Then, by subjecting the crude product to distillation under reduced pressure, 0.6 g of a purified product was obtained - boiling point 106°C/0.9 mmHg. (s)-(-)-1-benzyl-3-hydroxypyrrolidine thus obtained was 30% e. e. analyzed by the nuclear magnetic resonance spectra with respect to the proton at the 3-position using a shift reagent Eu-TFMC(III) .

Production of Diastereoisomer A

Example 1.

In 25 ml of chloroform was dissolved 4.91 g of the crude free base of YM-09730 obtained in Reference Example 1 and after adding thereto 15 ml of 10% hydrochloric acid followed by stirring well, the organic phase thus formed was separated. The organic layer was treated again in the above manner with 10 ml of 10% hydrochloric acid, and after drying the treated product with anhydrous magnesium sulfate, the solvent was distilled off under reduced pressure. The residue thus obtained was dissolved in 10.4 ml of acetone and the solution was allowed to stand to provide 3.5 g of the hydrochloride of YM-09730 as crystals. The product was disssolved in 1.8 ml of methanol and recrystallized with the addition of 8 ml of acetone. By repeating once, 2.38 g of the hydrochloride of YM-09730 was obtained. The ratio of diastereoisomer A : diastereoisomer B in the hydrochloride was 65.6 : 34.4 by HPLC. In 25 ml of chloroform was dissolved 2.15 g of the aforesaid salt, the solution thus obtained was washed twice each time with 15 ml of a saturated aqueous sodium hydrogencarbonate solution, and the organic layer thus formed was collected and dried over anhydrous magnesium sulfate. Then the solvent was distilled off under reduced pressure and 2 g of free base thus obtained was subjected to silica gel column chromatography (column-LiChroprep$^R$ Si60, C size; Eluent - ethyl acetate/acetic acid, 30 : 5 v/v) to provide the oily acetate of diastereoisomer A.

The product was dissolved in 10 ml of chloroform,and the solution thus formed was washed in succession with 10 ml of a saturated aqueous sodium hydrogencarbonate solution, 10 ml of water, and 10 ml of 10% hydrochloric acid and dried with anhydrous magnesium sulfate. Then the solvent was distilled off under reduced pressure and the residue thus obtained was treated with 0.8 ml of acetone to provide 0.4 g of the hydrochloride of diastereoisomer A.

Example 2

In 15 ml of acetonitrile were dissolved 4.91 g of the crude free base of YM-09730 obtained in Reference Example 1 and 1.04 g of malonic acid and the solution thus obtained was allowed to stand overnight at 0 to 5°C. Crystals thus formed were collected by filtration, washed with a small amount of cold acetonitrile to provide 2.03 g of the malonate of YM-09730. (Ratio of diastereoisomer A : B was 89.1 : 10.9). The product was recrystallized twice,each time from 25 times by volume of methanol,to provide 1.0g of the malonate of 100% diastereoisomer A of YM-09730.

Example 3.

In 15 ml of methanol were dissolved 4.91 g of the crude free base of YM-09730 obtained in Reference Example 1 and 1.04 g of malonic acid by heating and the solution thus formed was allowed to stand overnight at 0 to 5°C. Crystals thus deposited were collected by filtration, washed with methanol, and dried under reduced pressure to provide 1.88 g of the malonate of YM-09730. The ratio of diastereoisomers A : B of the crystals thus obtained was 90.7 : 9.3. The crystals were recrystallized twice from methanol to provide the malonate of diastereoisomer A containing no isomer B.

Example 4.

By following the same procedure as in Example 3 using acetonitrile in place of methanol, 2.03 g of a malonate was obtained. The ratio of diastereoisomers A : B was 89.1 : 10.9. The product was recrystallized from 25 times by volume of methanol to provide 1.57 g of the malonate of diastereoisomers A and B at a ratio of 99.5 : 0.5. Furthermore, by recrystallizing the product from 25 times by volume of methanol, 1.27 g of the maolonate of YM-09730(wherein the presence of diastereoisomer B was not detected by high performance liquid chromatography)was obtained. In 5 ml of chloroform was suspended 1.27 g of the malonate; the chloroform suspension was treated,in succession, twice each time with a saturated aqueous

sodium carbonate solution, twice each time with 2.5 ml of water, and then twice each time with 2.5 ml of 10% hydrochloric acid; the chloroform solution thus washed was dried over anhydrous magnesium sulfate, filtered, and evaporated to dryness under reduced pressure. The residue thus formed was dissolved in 2 ml of acetone and the solution was allowed to stand to deposit 1.09 g of the hydrochloride of diastereoisomer A of YM-09730.

Example 5.

In 5 ml of chloroform was suspended 595 mg of the malonate of distereoisomer A of YM-09730 and the suspension thus obtained was treated twice each time with 2.5 ml of a saturated aqueous sodium hydrogencarbonate solution and then twice each time with 5 ml of water. The chloroform solution thus obtained was dried over anhydrous magnesium sulfate and evaporated to dryness under reduced pressure to provide 491 mg of the free base of diastereoisomer A as a caramel . The product and 126 mg of oxalic acid dihydrate were dissolved in 3 ml of acetone, the solution thus formed was allowed to stand at 4°C, and the crystals thus deposited were collected by filtration to provide 400 mg of the oxalate of diastereoisomer A of YM-09730.

Example 6.

By following the Ex.5 procedure using 146 mg of 2-ketoglutaric acid in place of oxalic acid, 250 mg of the 2-ketoglutarate of diastereoisomerA of YM-09730 was obtained.

Example 7.

By following the Ex.5 procedure using 167 mg of p-nitrobenzoic acid in place of oxalic acid, 530 mg of p-nitrobenzoate of diastereoisomer A of YM-09730 was obtained.

Example 8.

By following the same procedure as in Example 5 using 116 mg of maleic acid in place of oxalic acid, 300 mg of the maleate of diastereoisomer A of YM-09730 was obtained.

Example 9.

In 2 ml of acetone was dissolved 491 mg of diastereoisomer A of YM-O973O and after adding thereto 1 ml of a methanol solution of 1 mole of phosphoric acid, the solution was allowed to stand at 4°C. Crystals thus deposited were collected by filtration to provide 480 mg of the phosphate of diastereoisomer A of YM-09730.

The properties of the desired compounds obtained in Examples 1 to 9 are shown in the following table.

| Product Compound | Formula | m.p. | C(%) | H(%) | N(%) | Cl(%) |
|---|---|---|---|---|---|---|
| | | | Properties: Elemental analysis | | | |
| Phosphate | $C_{27}H_{29}N_3O_6 \cdot H_3PO_4 \cdot 1/2H_2O$ | 216-218 | 54.39<br>54.18 | 5.56<br>5.56 | 6.96<br>7.02 | |
| Hydrochloride | $C_{27}H_{29}N_3O_6 \cdot HCl$ | 203-205 (decomp.) | 61.59<br>61.42 | 5.71<br>5.73 | 8.08<br>7.96 | 6.90<br>6.71 |
| p-Nitrobenzoate | $C_{27}H_{29}N_3O_6 \cdot C_7H_5NO_4 1/4H_2O$ | 150-151 | 61.58<br>61.58 | 5.20<br>5.24 | 8.32<br>8.32 | |
| Maleate | $C_{27}H_{29}N_3O_6 \cdot C_4H_4O_4$ | 168-169 | 61.40<br>61.28 | 5.49<br>5.47 | 6.85<br>6.92 | |
| 2-Ketoglutarate | $C_{27}H_{29}N_3O_6 \cdot C_5H_4O_3$ | 160-161 | 60.36<br>60.28 | 5.48<br>5.53 | 6.52<br>6.59 | |
| Oxalate | $C_{27}H_{29}N_3O_6 \cdot C_2H_2O_4$ | 179-180 | 60.14<br>60.36 | 5.62<br>5.70 | 6.93<br>6.88 | |
| Malonate | $C_{27}H_{29}N_3O_6 \cdot C_3H_4O_4$ | 181.5-182.5 (decomp.) | 59.89<br>60.50 | 5.37<br>5.58 | 7.23<br>7.06 | |

(Upper: Found values)
(Lower: Calculated values)

Hydrochloride: NMR (in $CD_3OD$, TMS internal standard, δppm)

1.80-2.70 (2H, broad m, $C_4'-H_2$)

2.32,2.34 (6H, s , $C_{2,6}-CH_3$)

3.0-4.0 (4H, m, $C_{2',5'}-H_2$)

3.63 (3H, s $-COOCH_3$)

4,40 (2H, s , $-CH_2-\phi$ )

5.08 (1H, s , $C_4-H$)

5.30 (1H, m, $C_{3'}-H$)

7.30-8.20 (9H, m, H of benzene ring)

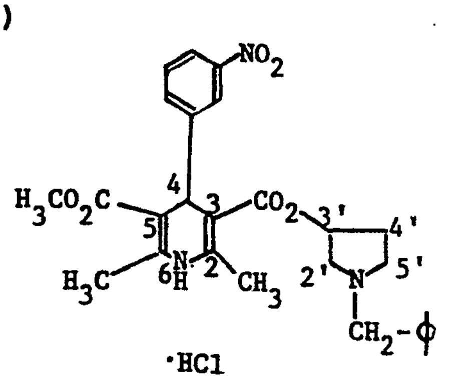

·HCl

Example 10

In 5 ml of chloroform was suspended 1.5 g of the malonate of diastereoisomer A obtained in Example 3 or 4 and the suspension was treated, in succession, twice each time with 3 ml of a saturated aqueous sodium hydrogencarbonate solution and then twice each time with 3 ml of water. The chloroform solution was dried over anhydrous magnesium sulfate and then the solvent was distilled off under reduced pressure. To the residue thus obtained was added 6 ml of ethanol and the mixture was allowed to stand overnight at 5°C to obtain O.86 g of crystals of the free base of diastereoisomer A of YM-09730.
Melting point 145 to 148°C

Elemental analysis for ($C_{27}H_{29}N_3O_6$)

| | C(%) | H(%) | N(%) |
|---|---|---|---|
| Calculated : | 65.98 | 5.95 | 8.55 |
| Found : | 66.04 | 6.00 | 8.53 |

NMR (in $CDCl_3$, TMS internal standard, δppm):

1.40 to 2.96 (6H, m, $C_{2',4',5'}$-$H_2$)

2.34, 2.36 (6H, s, $C_{2,6}$-$CH_3$)

3.65 (5H, s, -$COOCH_3$ and -$CH_2\phi$)

5.10 (1H, s, $C_4$-H)

5.12 (1H, m, $C_{3'}$-H)

5.78 (1H, broad s, NH)

7.18 to 8.25 (9H, m, H of benzene ring)

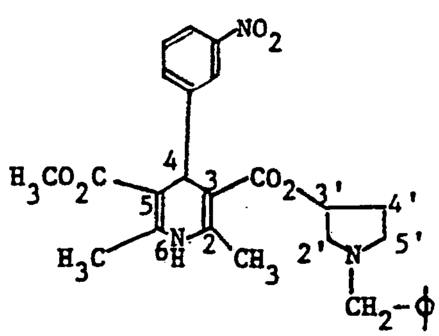

Production of dextro-rotatory isomer A

Example 11

(1) In 15 ml of acetonitrile were dissolved 4.91 g of the crude free base obtained in Reference Example 1 above and 1.04 g of malonic acid and the solution was allowed to stand overnight at 0 to 5°C. Crystals thus deposited were collected by filtration (2.03g) and recrystallized twice each time from 25 times by volume of methanol to provide 1.27 g of the malonate of diastereoisomer A of YM-0973O containing no diastereoisomer B. The melting point thereof was 181.5°C to 182.5°C (decomp.). In 5 ml of chloroform was suspended 1.27 g of the malonate thus obtained and the suspension was washed, in succession, twice each time with 2.5 ml of a saturated aqueous sodium hydrogencarbonate solution, once with 2.5 ml of water, and then twice each time with 2.5 ml of an aqueous 10% hydrochloric acid solution. The chloroform solution was dried over anhydrous magnesium sulfate and evaporated to dryness under reduced pressure. The residue thus formed was dissolved in 2 ml of acetone, the solution was allowed to stand, and 1.09 g of the hydrochloride of diastereoisomer A of YM-09730 thus deposited was collected.

(2) By treating 4.67 g of this hydrochloride of diastereoisomer A of YM-09730

with saturated aqueous sodium hydrogencarbonate, 4.35 g of the free base thereof was obtained and then 4.35 g of the free base thus obtained and 1.18 g of L-(-)-malic acid were dissolved in 28 ml of

ethanol with heating and the solution was allowed to stand overnight at 0 to 5°C. Crystals thus deposited were collected by filtration and dried to provide 2.43 g of the L-(-)-malate of the dextro-rotatory optical isomer of diastereoisomer A of YM-0973O. The crystals were recrystallized from 85 ml of ethanol to provide 2.21 g of the L-(-)-malate. The specific rotation$[\alpha]_D^{20}$ was + 82.2° (c = 0.5, methanol). When the product was recrystallized from ethanol, no change of specific rotation was observed.
Melting point 190 to 191°C (decomp.).

Elemental analysis (for $C_{27}H_{29}N_3O_6.C_4H_6O_5$)

| | C(%) | H(%) | N(%) |
|---|---|---|---|
| Found: | 59.72 | 5.80 | 6.73 |
| Calculated: | 59.51 | 5.64 | 6.72 |

Example 12

In 25 ml of acetone were dissolved 4.91 g of the crude free base of YM-09730 obtained in Reference Example 1 and 1.34 g of L-(-)-malic acid and the solution thus formed was stirred for 48 hours at 0 to 5°C. Crystals thus deposited were collected by filtration and washed with a small amount of cold acetone to provide 0.57 g of the L-(-)-malate of the dextro-rotatory optical isomer of diastereoisomer A of YM-09730. The specific rotation$[\alpha]_D^{20}$ was +78.3° (c = 0.5, methanol).

The specific rotation$[\alpha]_D^{20}$ of 0.44 g of the crystals obtained by recrystallizing the product from 50 times by volume of ethanol was +82.2° (c=0.5, methanol).
Melting point 190 to 191°C (decomp.).

Example 13

In 8 ml of chloroform was suspended 2.21 g of the L-(-)-malate of the dextro-rotatory optical isomer of diastereoisomer A of YM-0973O obtained in Example 11 and the suspension was treated, in succession, twice each time with 4.3 ml of a saturated aqueous sodium hydrogencarbonate solution, once with 4.3 ml of water, and then twice each time with 4.3 ml of 10% hydrochloric acid. The chlorofrom solution was dried over anhydrous magnesium sulfate and after filtration the solution was evaporated to dryness under reduced pressure. The residue thus formed was dissolved in 3.5 ml of acetone and the solution thus formed was allowed to stand to provide 1.64 g of the hydrochloride of the dextro-rotatory optical isomer of diastereoisomer A of YM-09730 as deposits.

The specific rotation$[\alpha]_D^{20}$ was + 116.5° (c = 0.5, methanol).
Melting point: 223 to 225°C (decomp.)

Elementary analysis (for $C_{27}H_{29}N_3O_6.HCl$)

| | C(%) | H(%) | N(%) | Cl(%) |
|---|---|---|---|---|
| Found: | 61.35 | 5.55 | 8.01 | 6.96 |
| Calculated: | 61.42 | 5.73 | 7.96 | 6.71 |

NMR (in $CD_3OD$ , TMS internal standard, δ ppm):

1.80-2.70 (2H, broad m, $C_{4'}-H_2$)

2.32, 2.34 (6H, s, $C_{2,6}-CH_3$)

3.0-4.0 (4H, m, $C_{2',5'}-H_2$)

3.64 (3H, s, $-COOCH_3$)

4.42 (2H, s, $-CH_2\phi$)

5.08 (1H, s $C_4-H$)

5.30 (1H, m, $C_{3'}-H$)

7.30-8.20 (9H, m, H of benzene ring)

Example 14

In 5 ml of isopropanol were dissolved (S)-3-acetoacetoxy-1-benzylpyrrolidine(obtained by reacting 1.77 g of (S)-(-)-1-benzyl-3-hydroxypyrrolidine ($[\alpha]_D^{20}$ -3.77°, c = 5, methanol) and 0.84 g of diketene for 3 hours at 70 to 80°C),1.51 g of m-nitrobenzaldehyde and 1.15 g of 3-aminocrotonic acid methyl ester,and the solution was refluxed for 8 hours. The solvent was then distilled off under reduced pressure.

The residue thus formed was dissolved in chloroform; the solution thus formed was washed in succession with dilute hydrochloric acid, water, and then a saturated aqueous sodium hydrogen carbonate solution, and dried over anhydrous magnesium sulfate. Then the solvent was distilled off under reduced pressure to provide 4.91 g of a mixture of the dextro-rotatory optical isomer of diastereoisomer A and the levo-rotatory optical isomer of diastereoisomer B of YM-09730 as a caramel. The crude free base thus obtained was subjected to silica gel column chromatography (column - Wakogel C-200, 2,000 g; eluent - ethyl acetate/acetic acid, 6 : 1 v/v), to obtain the oily acetate of the dextro-rotatory optical isomer of diastereoisomer A of YM-09730,showing a retention time of 28 min. by high-performance liquid chromatography.

The product was treated in chloroform with saturated aqueous sodium hydrogen carbonate and then dilute hydrochloric acid to provide 1.68 g of the hydrochloride of the dextro-rotatory optical isomer of diastereoisomer A of YM-09730. The specific rotation [$[\alpha]_D^{20}$ was + 116.5° (c = 0.5, methanol).

Example 15

In 25 ml of chloroform was dissolved 4.9 g of the crude free base obtained as in Example 14 and after adding thereto 15 ml of 10% hydrochloric acid, the resultant mixture was stirred well. Then the organic layer thus formed was separated, washed again with 10 ml of 10% hydrochloric acid, and dried over anhydrous magnesium sulfate. Thereafter, the solvent was distilled off under reduced pressure, the residue thus formed was dissolved in 10 ml of acetone, and the solution was allowed to stand for 2 days at 4°C to provide 2.7 g of the hydrochloride by collecting the crystals thus deposited. For removing the levo-rotatory optical isomer of diastereoisomer B therefrom, the product thus obtained was treated with saturated aqueous sodium hydrogencarbonate in chloroform to form a free base and after adding 0.68 g of L-(-)-malic acid in 15 ml of ethanol, the resultant mixture was allowed to stand for 2 days at 4°C. Crystals thus deposited were collected by filtration and recrystallized from ethanol to provide 1.33 g of the L-(-)-malate of the dextro-rotatory optical isomer of diastereoisomer A of YM-O973O showing a retention time of 28 min. by high-performance liquid chromatography. The specific rotation $[\alpha]_D^{20}$ was + 82.1° (c = 0.5, methanol).

Example 16

In 5 ml of chloroform was suspended 1.25 g of the L-(-)malate of the dextro-rotatory optical isomer of

diastereoisomer A of YM-09730 and the suspension thus obtained was treated, in succession, twice each time with 3 ml of saturated aqueous sodium hydrogen carbonate and then twice each time with 3 ml of water. The chloroform solution was dried over anhydrous magnesium sulfate and then the solvent was distilled off under reduced pressure. To the residue thus formed was added 6 ml of ethanol and the mixture was allowed to stand overnight at 5°C to provide 0.84 g of crystals of the free base of the dextro-rotatory optical isomer of diastereoisomer A of YM-09730.
Melting point 138 to 140°C.

Elemental analysis (for $C_{27}H_{29}N_3O_6$)

| | C(5) | H(%) | N(%) |
|---|---|---|---|
| Calculated: | 65.98 | 5.95 | 8.55 |
| Found: | 66.04 | 5.96 | 8.51 |

NMR (in $CDCl_3$, TMS internal standard, $\delta$ ppm)

| | |
|---|---|
| 1.40 to 3.0 | (6H, m, $C_{2',4'5'}-H_2$) |
| 2.34, 2.36 | (6H. s, $C_{2,6}-CH_3$) |
| 3.65 | (5H, s, $-COOCH_3$ and $-CH_2\phi$) |
| 5.10 | (1H, s, $C_4$-H) |
| 5.12 | (1H, m, $C_{3'}$-H) |
| 5.78 | (1H, broad s, -NH) |
| 7.16 to 8.24 | (9H, m, H of benzene ring). |

Formulation Example 1 (Tablet)

| | One tablet | 5,000 tabs. |
|---|---|---|
| Isomer A hydrochloride | 10.0 mg | 50 g |
| Lactose | 101.0 mg | 505 g |
| Corn starch | 25.3 mg | 126.5 g |
| Hydroxypropyl cellulose | 3.0 mg | 15 g |
| Magnesium stearate | 0.7 mg | 3.5 g |
| | 140 mg | 700 g |

To a uniform mixture of 50 g of the hydrochloride of isomer A, 505 g of lactose, and 126.5 g of corn starch was added 150 g of an aqueous 10% hydroxypropyl cellulose and the mixture was kneaded and

granulated. After drying, 3.5 g of magnesium stearate was added to the granules and they were uniformly mixed and then formed into tablets each of 140 mg.

Formulation Example 2 (Capsule)

| | One capsule | 1,000 cap. |
|---|---|---|
| Isomer A hydrochloride | 10.0 mg | 10 g |
| Crystal lactose | 189.0 mg | 189 g |
| Magnesium stearate | 1.0 mg | 1 g |
| | 200 mg | 200 g |

The above components were uniformly mixed and 200 mg thereof was filled into each capsule to provide capsule medicaments.

Formulation Example 3 (Injection)

| | |
|---|---|
| Isomer A hydrochloride | 1 mg |
| D-sorbitol | 100 mg |

The above components were dissolved in distilled water for injection and after adjusting the pH thereof to 4 by the addition of hydrochloric acid, distilled water for injection was added to make the total volume to 2 ml.

**Claims**

1. Diastereoisomer A of 2,6-dimethyl-4-(3-nitrophenyl)-1,4-dihydropyridine-3,5-dicarboxylic acid 3-(1-benzylpyrrolidin-3-yl) ester 5-methyl ester, the melting point of the hydrochloride of said diastereoisomer A being 200 to 206°C(decomp.), or the dextro-rotatory optical isomer of this diastereoisomer A, or a pharmaceutically acceptable acid addition salt of said diastereoisomer or of said dextro-rotatory optical isomer.

2. An acid addition salt according to claim 1 which is a malonate, oxalate, p-nitrobenzoate 2-ketoglutarate, maleate, phosphate, hydrochloride, sulfate, or p-toluenesulfonate.

3. A process of producing a compound according to claim 1 which comprises subjecting a mixture of diastereoisomers A and B of 2,6-dimethyl-4-(3-nitrophenyl)-1,4-dihydropyridine-3,5-dicarboxylic acid 3-(1-benzylpyrrolidin-3-yl) ester 5-methyl ester to column chromatography using silica gel as a carrier and a mixture of ethyl acetate and acetic acid as an eluent, and taking the eluted acetate of diastereoisomer A.

4. A process of producing a compound according to claim 1 which comprises fractionally recrystallizing a mixture of an acid addition salt of diastereoisomers A and B of 2,6-dimethyl-4-(3-nitrophenyl)-1,4-dihydropyridine-3,5-dicarboxylic acid 3-(1-benzylpyrrolidin-3-yl) ester 5-methyl ester to provide the salt corresponding to diastereoisomer A.

5. A process as claimed in claim 4, wherein an acid addition salt to be fractionally recrystallized is malonate, p-nitrobenzoate or maleate.

6. A process as claimed in claim 3, 4 or 5 including treating the resulting salt with base to give the diastereoisomer A.

7. A process as claimed in claim 6 including treating the resulting diastereoisomer A with pharmaceutically acceptable acid.

8. The L-(-)-malate or the hydrochloride of the dextro-rotatory optical isomer of diastereoisomer A of claim 1.

9. A process of producing a compound according to claim 1 which comprises producing diastereoisomer A by a process according to claim 3 or 4 or 5 and then reacting resulting diastereoisomer A with L-(-)-malic acid to deposit the L-(-)-malate of the dextro-rotatory optical isomer of diastereoisomer A.

10. A process of producing a compound according to claim 1 which comprises providing a mixture (of formula I below) of the dextro-rotatory optical isomer of diastereoisomer A and the levo-rotatory optical isomer of diastereoisomer B of 2,6-dimethyl-4-(3-nitrophenyl)-1,4-dihydropyridine-3,5-dicarboxylic acid 3-(1-benzylpyrrolidin-3-yl) ester 5-methyl ester

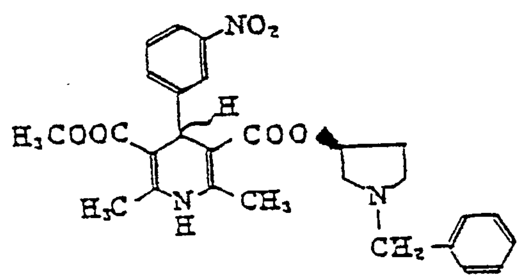

(I)

(wherein the bond shown by the wavy line represents an α or β bond and the bond shown by the dense arrow represents a β bond), and (a)subjecting the mixture to column chromatography using silica gel as a carrier and a mixture of ethyl acetate and acetic acid as an eluent to separate the acetate of the dextro-rotatory optical isomer of diastereoisomer A, or (b) reacting said mixture with L-(-)-malic acid to deposit the L-(-)-malate of the dextro-rotatory optical isomer of diastereoisomer A.

11. A process according to claim 10 wherein the formula I mixture is obtained by :
(i) reacting m-nitrobenzaldehyde represented by formula (II)

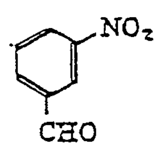

(II),

(S)-3-acetoacetoxy-1-benzylpyrrolidine represented by formula (III)

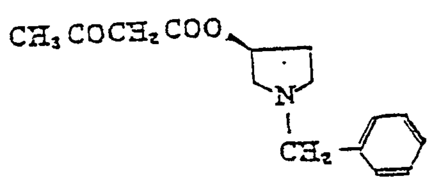

(III),

(wherein the bond shown by the dense arrow represents a β bond), and 3-aminocrotonic acid methyl ester represented by formula (IV)

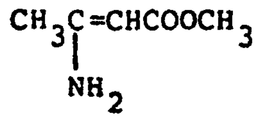

(IV); or

(ii) reacting m-nitrobenzaldehyde represented by formula (II) above, acetoacetic acid methyl ester represented by formula (V)

$CH_3COCH_2CQOCH_3$ (V),

and (S)-3-(3-aminocrotonoyloxy)-1-benzylpyrrolidine represented by formula (VI)

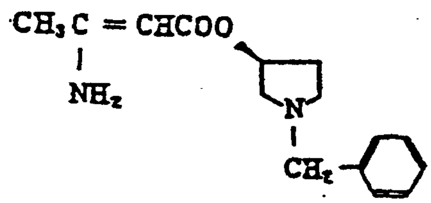

(VI); or

(iii) reacting (S)-1-benzyl-3-[2-m-nitrobenzylidene)acetoacetoxy]pyrrolidine(represented by formula (VII)

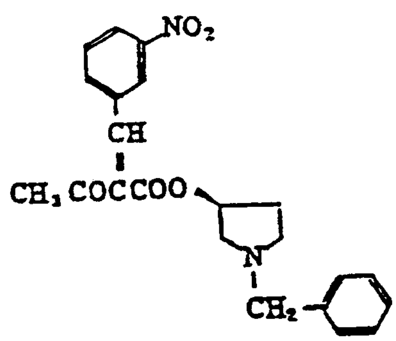

(VII)

and
obtained by previously reacting m-nitrobenzaldehyde shown by formula (II) above and (S)-3-acetoacetoxy-1-benzylpyrrolidine shown by formula (III) above) with 3-aminocrotonic acid methyl ester represented by formula (VI) above; or
(iv) reacting 2-(m-nitrobenzylidene)acetoacetic acid methyl ester (represented by formula (VIII)

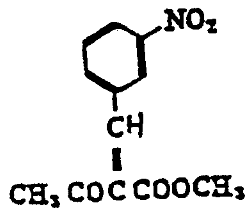

(VIII)

and
obtained by previously reacting m-nitrobenzaldehyde shown by formula (II) above and acetoacetic acid methyl ester shown by formula (V) above) with
(S)-3-(3-aminocrotonoyloxy)-1-benzylpyrrolidine represented by formula (VI) above.

12. A process as claimed in claim 9 or 10 or 11 including treating the resulting acetate or L-(-)-malate with base to give the dextro-rotatory optical isomer of diastereoisomer A.

13. A process according to claim 12 including treating the resulting dextro-rotatory optical isomer with pharmaceutically acceptable acid.

14. A pharmaceutical composition comprising an effective amount of at least one compound according to claim 1.

## Revendications

1. Diastéréoisomère A de 1,4-dihydro-2,6-diméthyl-3-carboxylate de (1-benzylpyrrolidin-3-yl)-5-carboxylate de méthyle-4-(m-nitrophényl) pyridine, le point de fusion du chlorhydrate dudit diastéréoisomère étant de 200 à 206°C (décomp.) ou l'isomère optique dextrogyre de ce diastéréoisomère A, ou un sel d'addition acide pharmaceutiquement acceptable dudit diastéréoisomère ou dudit isomère optique dextrogyre.

2. Un sel d'addition acide conforme à la revendication 1 qui est un malonate, un oxalate, un p-nitrobenzoate, un 2-cétoglutarate, un maléate, un phosphate, un chlorhydrate, un sulfate ou un p-toluène-sulfonate.

3. Un procédé de production d'un compose conforme à la revendication 1 qui comprend la soumission d'un mélange des diastéréoisomères A et B de 1,4-dihydro-2,6-diméthyl-3-carboxylate de (1-benzylpyrrolidin-3-yl)-5-carboxylate de méthyle-4-(m-nitrophényl) pyridine à une chromatographie colonne utilisant un gel de silice comme support et un mélange d'acétate d'éthyle et d'acide acétique comme éluant et en prenant l'acétate élué du diastéréoisemère A.

4. Un procédé de production d'un composé conforme à la revendication 1, qui consiste à recristalliser de manière fractionnelle un mélange d'un sel d'addition acide de diastéréoisomères A et B de 1,4-dihydro-2,6-diméthyl-3-carboxylate de (1-benzylpyrrolidin-3-yl)-5-carboxylate de méthyle-4-(m-nitrophényl) pyridine pour former le sel correspondant au diastéréoisomère A.

5. Un procédé tel que revendiqué dans la revendication 4, dans lequel un sel d'addition acide à recristalliser par fractionnement est un malonate, un p-nitrobenzoate, ou un maléate.

6. Un procédé tel que revendiqué dans l'une des revendications 3, 4 ou 5 incluant le traitement du sel résultant avec une base pour donner le diastéréoisomère A.

7. Un procédé tel que revendiqué dans la revendication 6 incluant le traitement du diastéréoisomère A résultant avec un acide pharmaceutiquement acceptable.

8. Le L-(-)-maléate ou le chlorhydrate de l'isomère optique dextrogyre du diastéréoisomère A de la revendication 1.

9. Un procédé de production d'un composé conforme à la revendication 1 qui consiste à produire un diastéréoisomère A par un procédé conforme aux revendications 3, 4 ou 5 et ensuite à faire réagir le diastéréoisomère résultant A avec l'acide L-(-)-malique pour former un dépôt de L-(-)-malate de l'isomère optique dextrogyre du diastéréoisomère A.

10. Un procédé de production d'un composé conforme à la revendication 1 qui consiste à fournir un mélange (de formule I ci dessus) de l'isomère optique dextrogyre du diastéréoisomère A et de l'isomère optique lévogyre du diastéréoisomère B de 1,4-dihydro 2,6-diméthyl-3-carboxylate de (1-benzylpyrrolidin-3-yl)-5-carboxylate de méthyle-4-(m-nitrophényl) pyridine,

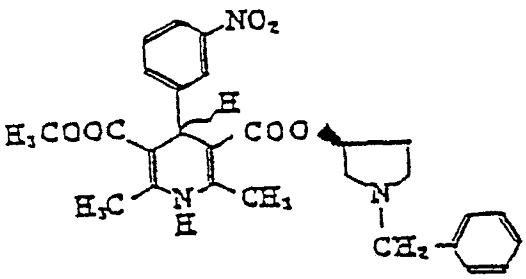

(I)

(dans lequel la liaison représentée par la ligne ondulée représente une liaison α ou β et la liaison représentée par la flèche en gras représente une liaison β), et (a) à soumettre le mélange à une chromatographie colonne en utilisant un gel de silice comme support et un mélange d'acétate d'éthyle et d'acide acétique comme éluant pour séparer l'acétate de l'isomère optique dextrogyre du diastéréoisomère A, ou (b) à faire réagir ledit mélange avec l'acide L-(-)-malique pour former un dépôt de L-(-)-malate de l'isomère optique dextrogyre du diastéréoisomère A.

**11.** Un procédé conforme à la revendication 10 dans lequel le mélange de formule I est obtenu par :
(i) la réaction du m-nitrobenzaldéhyde représenté par la formule (II)

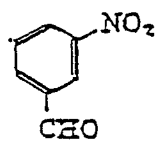

(II),

(S)-3-acétoacétoxy-1-benzylpyrrolidine représenté par la formule (III)

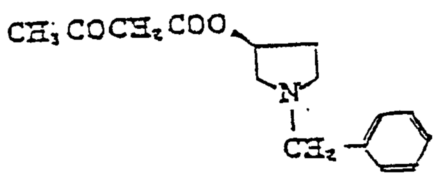

(III),

(dans laquelle la liaison représentée par la flèche en gras représente une liaison β), et l'amino-3 crotonate de méthyle représenté par la formule (IV)

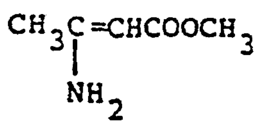

(IV);

ou
(ii) en faisant réagir le m-nitrobenzaldéhyde représenté par la formule (II) ci-dessus, l'acéthylacétate de méthyle représenté par la formule (V)

$CH_3COCH_2COOCH_3$ (V),

et la (S)-3-(3-aminocrotonoyloxy)-1-benzyl-pyrrolidine représentée par la formule (VI)

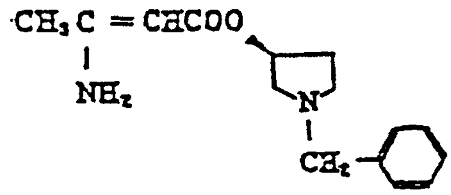

(VI),

ou

(iii) en faisant réagir la (S)-1-benzyl-3-[2-m-nitrobenzylidène) acétoacétoxy] pyrrolidine (représentée par la formule (VII)

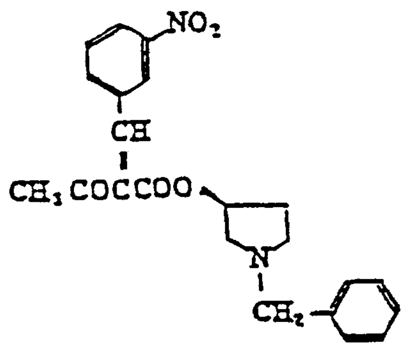

(VII)

et

obtenue en faisant réagir auparavant le m-nitrobenzaldéhyde représenté par la formule (II) ci-dessus et la (S)-3-acétoacétoxy-1-benzylpyrrolidine représentée par la formule (III) ci-dessus) avec l'amino-3 crotonate de méthyle représenté par la formule (VI) ci-dessus; ou

(iv) en faisant réagir le 2-(m-nitrobenzylidène) acétoacétate de méthyle (représenté par la formule (VIII)

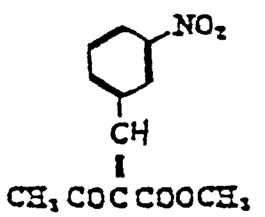

(VIII)

et obtenu en faisant réagir préalablement le m-nitrobenzaldéhyde représenté par la formule (II) ci-dessus et l'acétoacétate de méthyle représenté par la formule (V) ci-dessus) avec la (S)-3-(3-aminocrotonoyloxy)-1-benzylpyrrolidine représentée par la formule (VI) ci-dessus.

**12.** Un procédé tel que revendiqué dans les revendications 9 ou 10 ou 11 incluant le traitement de l'acétate résultant ou du L-(-)-malate avec une base pour donner l'isomère optique dextrogyre du diastéréoisomère A.

**13.** Un procédé conforme à la revendication 12 incluant le traitement de l'isomère optique dextrogyre résultant avec un acide pharmaceutiquement acceptable.

**14.** Une composition pharmaceutique comprenant une quantité efficace d'au moins un composé conforme à la revendication 1.

**Patentansprüche**

1. Diastereoisomer A von 2,6-Dimethyl-4-(3-nitrophenyl)-1,4-dihydropyridin-3,5-dicarbonsäure-3-(1-benzylpyrrolidin-3-yl)ester-5-methylester, wobei der Schmelzpunkt des Hydrochlorids dieses Diastereoisomers A 200 bis 206° C (Zers.) beträgt, oder das rechts-drehende optische Isomer dieses Diastereoisomers A, oder ein pharmazeutisch annehmbares Säureadditionssalz dieses Diastereoisomers oder dieses rechts-drehenden optischen Isomers.

2. Säureadditionssalz nach Anspruch 1, das ein Malonat, Oxalat, p-Nitrobenzoat, 2-Ketoglutarat, Maleat, Phosphat, Hydrochlorid, Sulfat oder p-Toluolsulfonat ist.

3. Verfahren zur Herstellung einer Verbindung nach Anspruch 1, das darin besteht, daß man ein Gemisch der Diastereoisomeren A und B von 2,6-Dimethyl-4-(3-nitrophenyl)-1,4-dihydropyridin-3,5-dicarbonsäure-3-(1-benzylpyrrolidin-3-yl)ester-5-methylester der Säulenchromatographie unterwirft unter Verwendung von Silicagel als Träger und eines Gemisches aus Ethylacetat und Essigsäure als Eluens, und das eluierte Acetat des Diastereoisomers A aufnimmt.

4. Verfahren zur Herstellung einer Verbindung nach Anspruch 1, umfassend das fraktionierte Umkristallisieren eines Gemisches eines Säureadditionssalzes der Diastereoisomeren A und B von 2,6-Dimethyl-4-(3-nitrophenyl)-1,4-dihydropyridin-3,5-dicarbonsäure-3-(1-benzylpyrrolidin-3-yl)ester-5-methylester, um das dem Diastereoisomer A entsprechende Salz zu erhalten.

5. Verfahren nach Anspruch 4, wobei das Säureadditionssalz, das fraktioniert umkristallisiert wird, Malonat, p-Nitrobenzoat oder Maleat ist.

6. Verfahren nach Anspruch 3, 4 oder 5, umfassend die Behandlung des erhaltenen Salzes mit einer Base unter Bildung des Diastereoisomers A.

7. Verfahren nach Anspruch 6, umfassend die Behandlung des erhaltenen Diastereoisomers A mit einer pharmazeutisch annehmbaren Säure.

8. L-(-)-Malat oder Hydrochlorid des rechts-drehenden optischen Isomers des Diastereoisomers A nach Anspruch 1.

9. Verfahren zur Herstellung einer Verbindung nach Anspruch 1, umfassend die Bildung des Diastereoisomers A nach einem Verfahren nach Anspruch 3 oder 4 oder 5, und anschließende Umsetzung des erhaltenen Diastereoisomers A mit L-(-)-Apfelsäure, um das L-(-)-Malat des rechts-drehenden optischen Isomers des Diastereoisomers A abzuscheiden.

10. Verfahren zur Herstellung einer Verbindung nach Anspruch 1, umfassend die Zurverfügungstellung eines Gemisches (der Formel I, wie unten) des rechts-drehenden optischen Isomers des Diastereoisomers A und des links-drehenden optischen Isomers des Diastereoisomers B von 2,6-Dimethyl-4-(3-nitrophenyl)-1,4-dihydropyridin-3,5-dicarbonsäure-3-(1-benzylpyrrolidin-3-yl)ester-5-methylester

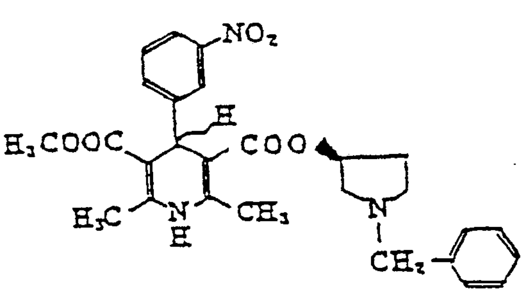

(I)

(wobei die durch die Wellenlinie angegebne Bindung eine $\alpha$- oder $\beta$-Bindung bezeichnet und die durch den Pfeil angegebene. Bindung eine $\beta$-Bindung bedeutet) und (a) das Gemisch der Säulenchromaphie unter Verwendung von Silicagel als Träger und einem Gemisch aus Ethylacetat und Essigsäure als Eluens unterwirft, um das Acetat des rechts-drehenden optischen Isomers des Diastereoisomers A

abzutrennen, oder (b) das Gemisch mit L-(-)-Apfelsäure umsetzt, um das L-(-)-Malat des rechtsdrehenden optischen Isomers des Diastereoisomers A zu erhalten.

**11.** Verfahren nach Anspruch 10, wobei das Gemisch der Formel I erhalten wird durch:
(i) Umsetzung von m-Nitrobenzaldehyd der Formel (II)

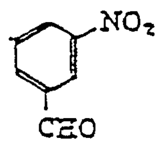

(II),

(S)-3-Acetoacetoxy-1-benzylpyrrolidin der Formel (III)

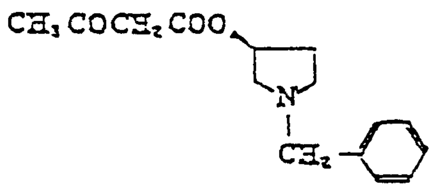

(III),

(wobei die durch den Pfeil angegebene Bindung eine $\beta$-Bindung bedeutet), und 3-Aminocrotonsäure-methylester der Formel (IV)

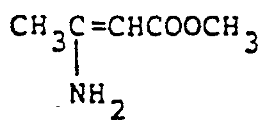

(IV); oder

(ii) Umsetzung von m-Nitrobenzaldehyd der obigen Formel (II), Acetessigsäure-methylester der Formel (V)

$CH_3COCH_2COOCH_3$ (V)

und (S)-3-(3-Aminocrotonoyloxy)-1-benzyl-pyrrolidin der Formel (VI)

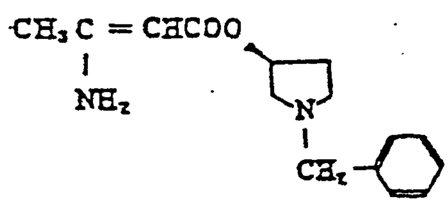

(VI); oder

(iii) Umsetzung von (S)-1-Benzyl-3-[2-m-nitrobenzylidin)acetoacetoxy]pyrrolidin (der Formel (VII)

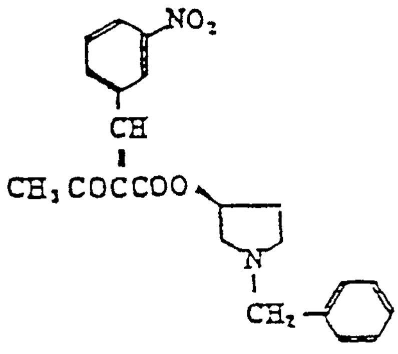

(VII)

und erhalten durch vorherige Umsetzung von m-Nitrobenzaldehyd der obigen Formel (II) und (S)-3-Acetoacetoxy-1-benzylpyrrolidin der Formel (III) oben) mit 3-Aminocrotonsäure-methylester der Formel (VI) oben; oder

(iv) Umsetzung von 2-(m-Nitrobenzyliden)acetessigsäure-methylester (der Formel (VIII)

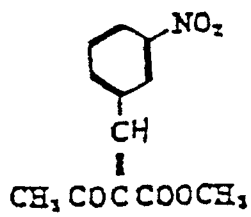

(VIII)

und erhalten durch vorherige Umsetzung von m-Nitrobenzaldehyd der Formel (II) oben und Acetessigsäure-methylester der Formel (V) oben) mit (S)-3-(3-Aminocrotonoyloxy)-1-benzylpyrrolidin der Formel (VI) oben.

**12.** Verfahren nach Anspruch 9 oder 10 oder 11, umfassend die Behandlung des erhaltenen Acetats oder L-(-)-Malats mit einer Base unter Bildung des rechts-drehenden optischen Isomers des Diastereoisomers A.

**13.** Verfahren nach Anspruch 12, umfassend die Behandlung des erhaltenen rechts-drehenden optischen Isomers mit einer pharmazeutisch annehmbaren Säure.

**14.** Pharmazeutisches Mittel, enthaltend eine wirksame Menge mindestens einer Verbindung nach Anspruch 1.